(19) Europäisches Patentamt
European Patent Office
Office européen des brevets

(11) **EP 1 774 907 A1**

(12) **EUROPEAN PATENT APPLICATION**

(43) Date of publication:
**18.04.2007 Bulletin 2007/16**

(51) Int Cl.:
**A61B 5/0456** (2006.01)  **G06F 17/00** (2006.01)

(21) Application number: **05256339.2**

(22) Date of filing: **12.10.2005**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(71) Applicant: **Jetfly Technology Limited
Kowloon, Hong Kong (CN)**

(72) Inventor: **Wong, Kin Kai Andy
New Territories
Hong Kong (HK)**

(74) Representative: **Howe, Steven
Lloyd Wise
Commonwealth House,
1-19 New Oxford Street
London WC2A 1LW (GB)**

(54) **Method and device for measuring heart rate**

(57) A method of measuring heart rate of an individual is disclosed as including the steps of (a) pre-setting a number of discrete detection thresholds including at least a highest threshold and a lowest threshold; (b) pre-setting a first condition for change of detection threshold; (c) detecting a stream of signals in an ECG waveform; (d) detecting QRS complexes in the ECG waveform by applying a first detection threshold; and (e) detecting QRS complexes in the ECG waveform by applying instead a second threshold which is different from the first detection threshold if the first condition is met in step (d).

Fig. 2

**Description**

This invention relates to a method and device for measuring heart rate, in particular such method and device suitable for measuring the heart rate of an individual by detecting QRS complexes of detected electrocardiographic (ECG) signals of the individual.

When a heart of an individual beats, electrical signals (called "ECG signals") flow through the heart, which may be picked up by appropriate devices attached to the skin of the individual. A typical and complete ECG signal includes a complete waveform, which is said to be comprised of a P wave, a Q wave, an R wave, an S wave and a T wave, in which the Q wave, R wave and S wave are sometimes collectively referred to as forming a "QRS complex". Generally speaking, the P-wave arises from the depolarisation of the atrium, the QRS complex arises from depolarisation of the ventricles, and the T wave arises from re-polarisation of the ventricle muscle. Generally, the R wave is the most prominent wave in the waveform of the whole ECG signal. The heart rate (or rate of heart beat) of an individual may thus be determined by detecting and measuring the time intervals between successive R waves of the ECG signals.

Various heart rate monitors have thus been provided for detecting such R waves or QRS complexes at the skin level for monitoring the heart rate of the individual wearing such devices, for sports and/or medical applications. The devices may be in the form of a chest belt worn around the chest of the individual, with electrodes placed on the skin close to the heart of the individual for picking up the ECG signals. Alternatively, the devices may be in the form of a wrist watch (whether with or without usual watch functions) worn around the wrist of the user, to pick up ECG signals *via* electrodes placed next on the wrist of the user.

However, ECG signal and its noise level vary from person to person. Even for the same person, they vary from time to time. Furthermore, some people have weak ECG signals, i.e. the QRS peak is very small. For people with strong ECG signal, they also have a high T wave which may be easily mistakenly identified as the R wave.

Although various methods and devices have been proposed for solving such a problem, most such methods and devices are very complicated, which adds to the complexity of the associated hardware or software, leading to an increase in the size of the circuit or an increase in the microcontroller (MCU) requirement.

It is thus an object of the present invention to provide a method and device for measuring heart rate of an individual in which the aforesaid shortcomings are mitigated, or at least to provide a useful alternative to the trade and public.

According to a first aspect of this invention, there is provided a method of measuring heart rate of an individual, including the steps of (a) pre-setting a plurality of discrete detection thresholds including at least a highest threshold and a lowest threshold; (b) pre-setting at least a first condition for change of detection threshold; (c) detecting a stream of signals in an ECG waveform; (d) detecting QRS complexes in said ECG waveform by applying a first one of said pre-set detection thresholds; and (e) detecting QRS complexes in said ECG waveform by applying instead a second one of said pre-set detection thresholds which is different from said first detection threshold if said at least first condition is met in said step (d).

According to a second aspect of this invention, there is provided a device for measuring heart rate of an individual, including means for pre-setting a plurality of discrete detection thresholds including at least a highest threshold and a lowest threshold; and means for detecting a stream of signals in an ECG waveform; wherein said device is adapted to detect QRS complexes in said ECG waveform by applying a first one of said pre-set detection thresholds; characterized in that said device is adapted to detect QRS complexes in said ECG waveform by applying instead a second one of said pre-set detection thresholds which is different from said first detection threshold if at least a first pre-set condition is met.

Embodiments of the present invention will now be described, by way of examples only, with reference to the accompanying drawings, in which:

Fig. 1 is a flow chart showing a first method of measuring heart rate according to the present invention;
Fig. 2 shows an exemplary ECG waveform as detected by the method of Fig. 1;
Fig. 3 is a functional block diagram of a first exemplary heart rate measuring device according to the present invention;
Fig. 4 is a functional block diagram of a second exemplary heart rate measuring device according to the present invention;
Fig. 5 is a functional block diagram of a software implementation of a heart rate measuring device according to the present invention;
Fig. 6 is a functional block diagram of a hardware implementation of a heart rate measuring device according to the present invention; and
Fig. 7 is a flow chart showing a second method of measuring heart rate according to the present invention.

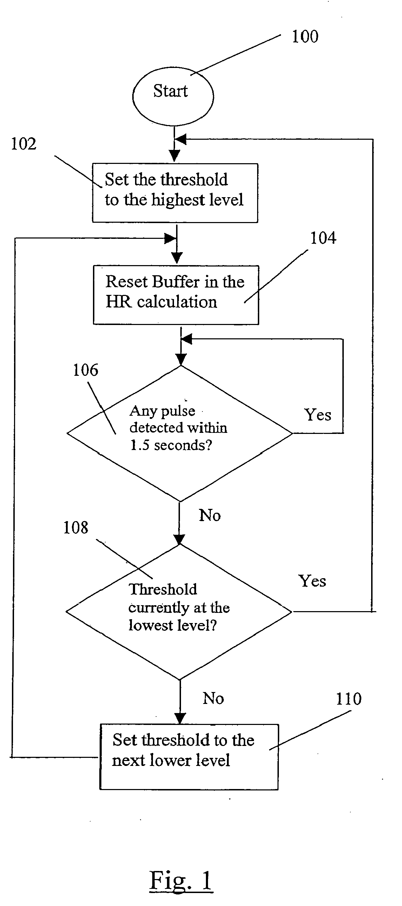

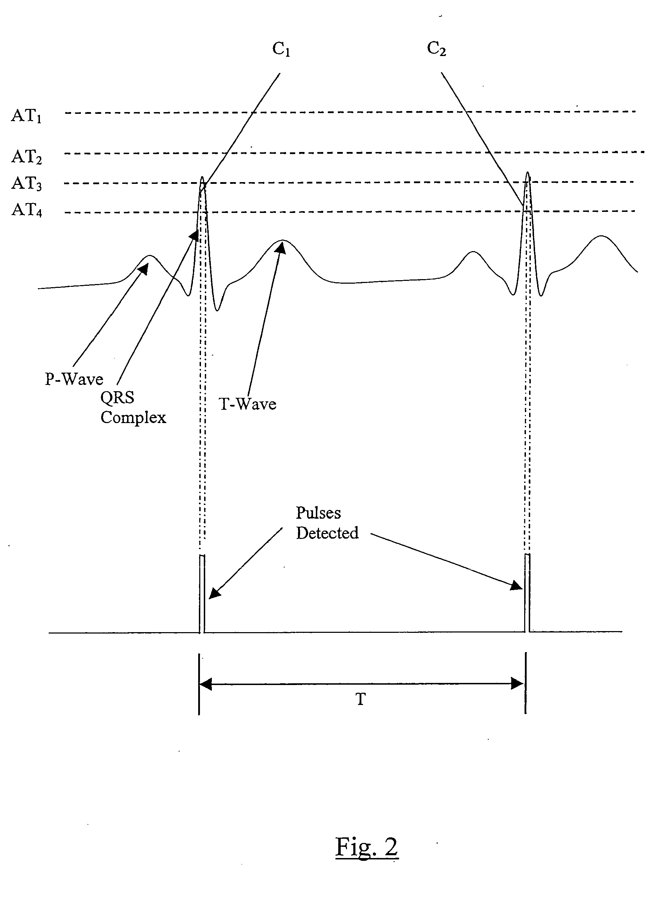

Referring to Figs. 1 and 2, in a method for measuring heart rate according to an embodiment of the present invention, a total of four discrete amplitude thresholds $AT_1$, $AT_2$, $AT_3$ and $AT_4$ are set for detecting QRS complexes in an ECG signal waveform.

When the method starts (100), the threshold for detecting QRS complexes is set at the highest level (102), i.e. $AT_1$. The buffer in the heart rate calculation circuit (HR calculation) is reset (104). Pulses, as represented by QRS complexes in the ECG signal waveform, are detected by applying threshold $AT_1$ so long as a pulse is detected within 1.5 seconds after the preceding pulse is detected. If no pulse is detected for any continuous time interval of 1.5 seconds (106), it is then checked whether pulses are being detected by the application of the lowest threshold (i.e. $AT_4$ in Fig. 2) (108). If it is not yet at the lowest threshold, the threshold for detecting pulses will be set to the next lower level (110). Thus, if the current threshold is $AT_1$, the threshold will be set to $AT_2$. If, however, the lowest threshold is already reached, the threshold will be set to the highest level $AT_1$ (102).

As shown in Fig. 2, with the waveform as shown, if the threshold is set at $AT_1$, no pulse will be detected within any 1.5-second time interval. The threshold will then be set to $AT_2$, the threshold level next lower to $AT_1$. As, again, no pulse will be detected within any 1.5-second time interval at threshold $AT_2$, the threshold will then be set to $AT_3$, the threshold level next below $AT_2$. At threshold $AT_3$, the QRS complexes $C_1$ and $C_2$ are detected, with a time interval of T. If time interval T is within 1.5 seconds, QRS complexes (and thus pulses) will continue to be detected at threshold $AT_3$. If no pulse (QRS complex) is detected for any 1.5-second time interval, the threshold will be set to the next lower level $AT_4$. If, at $AT_4$, which is the lowest threshold, it still exists the situation where no pulse (QRS complex) is detected for any 1.5-second time interval, the threshold will be set to the highest level $AT_1$.

The time interval T can be used for calculating the instantaneous heart rate (HR) of the individual, measured in beats per minute, according to the following Equation 1:

$$HR = \frac{60}{T} \quad \text{................................................} \quad Equation\ 1$$

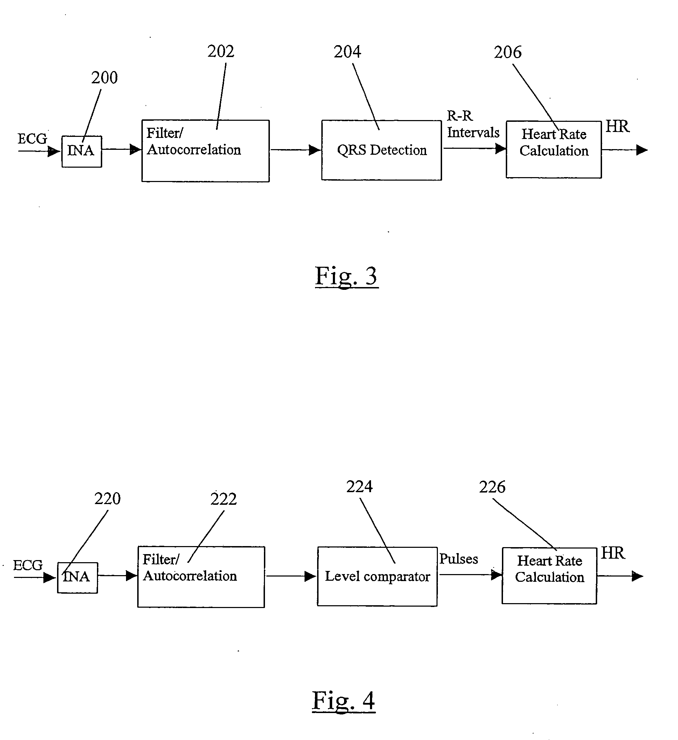

As shown in Fig. 3, a heart rate monitor adopting the above method includes an Instrumentation Amplifier (INA) (200) for amplifying the detected, incoming ECG signals, which are then passed to a set of filters and autocorrelation circuits (202) for removal of noise of the amplified signals. An QRS detection circuit (204) then processes the filtered signals for detection of QRS complexes by analyzing the features of QRS complexes in the ECG waveform. The time intervals between successive detected QRS complexes are then passed to a heart rate calculation circuit (206) for arriving at the instantaneous heart rate (HR), for subsequent output.

As shown in Fig. 4, a second heart rate monitor adopting the above method also includes an Instrumentation Amplifier (INA) (220) and a set of filters and autocorrelation circuits (222). The ECG signals so processed are then passed to a level comparator (224) which makes comparison on the amplitude of the waveform. The output from the level comparator (224) are then fed to a heart rate calculation circuit (226) for arriving at the instantaneous heart rate for subsequent output.

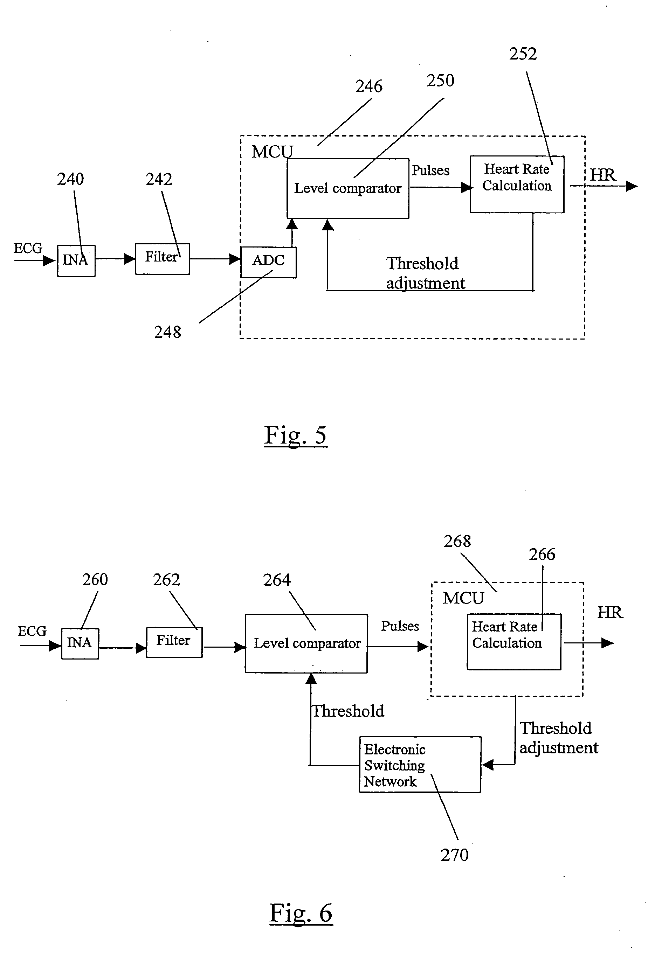

Fig. 5 shows a block diagram of the software implementation of the ECG peak detection with adaptive threshold. After passing the incoming ECG signals through an INA (240) and a filter circuit (242), the signals are then fed to a microcontroller (MCU) (246) for arriving at the heart rate (HR). The MCU (246) includes an analogue-to-digital converter (ADC) (248) which converts the ECG signals into digital signals for transmission to a level comparator (250). Detected pulses are then sent to a heart rate calculation circuit (252) for calculation of the heart rate. The heart rate calculation circuit (252) will send instructions to the level comparator (250) to change the threshold level for making the comparison if no pulse is received for any 1.5-second time interval.

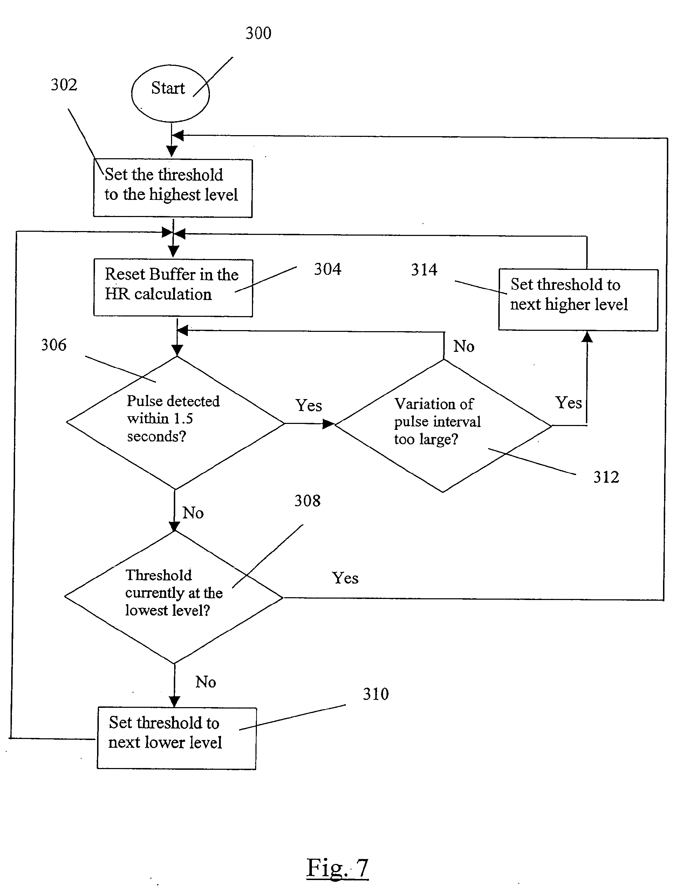

Fig. 6 shows a block diagram of the hardware implementation of the ECG peak detection with adaptive threshold. After passing the incoming ECG signals through an INA (260) and a filter circuit (262), the signals are then fed to a level comparator (264). Detected pulses are then sent to a heart rate calculation circuit (266) in an MCU (268) for calculation of the heart rate. If no pulse is received for any 1.5-second time interval, the MCU (268) will send instructions to an electronic switching network (270) which switches on and off the pre-set thresholds for adjusting the threshold for detecting pulses.

A flow chart showing an alternative method is shown in Fig. 7. When the method starts (300), the threshold for detecting QRS complexes is set to the highest level (302). The buffer in the heart rate calculation circuit (HR calculation) is reset (304). Pulses, which are represented by QRS complexes in the ECG signal waveform, are detected by applying the highest threshold. If no pulse is detected for 1.5 seconds (306), it is then checked if the lowest threshold is reached (308). If it is not yet in the lowest threshold, the threshold for detecting pulses will be set to the next lower level (310). If, however, the lowest threshold is already reached, the threshold will be set to the highest level (302).

During detection of pulses by applying a certain threshold, the extent of variation of the time intervals between successive pulses ("pulse interval") is continuously monitored (312). If the extent of variation is not above a pre-set value, the current threshold will continue to be used for detecting pulses. If, on the other hand, the extent of variation is above a pre-set value, the detection threshold will be set to the next higher level (314).

The extent of the variation of the pulse interval may be measured by the standard deviation of the pulse intervals. The standard deviation σ of pulse intervals $T_1$, $T_2$, ...$T_N$ may be obtained by the following Equation 2:

$$\sigma = \sqrt{\frac{1}{N}\sum_{i=1}^{N}(T_i - \overline{T})^2} \quad \dots\dots\dots\dots\dots\dots\dots\dots\dots\dots\dots\dots\dots \quad \text{Equation 2}$$

where $\overline{T}$ is the arithmetic mean of pulse intervals $T_1$, $T_2$, ... $T_N$, as given in the following Equation 3:

$$\overline{T} \;=\; \frac{1}{N}\sum_{i=1}^{N}T_i \dots\dots\dots\dots\dots\dots\dots\dots\dots\dots\dots\dots\dots\dots\dots \quad \text{Equation 3}$$

Thus, in this case, if the standard deviation of the detected pulse intervals $T_1$, $T_2$, ...$T_N$ is above a certain pre-set value, the threshold for detecting pulses will be set to the next above level.

As a further alternative method, the first detection threshold to be applied when starting the detection method may be the lowest threshold. In such a case, the extent of variation (e.g. as determined by the standard deviation) of the pulse intervals is constantly determined and compared with a pre-set value. If, say, the standard deviation is larger than the pre-set value, the detection threshold will be changed to the next one above, and so on until the standard deviation is smaller than the pre-set value. At the same time, it will also be checked as to whether any pulse (i.e. QRS complex) is detected within a continuous pre-determined time interval, e.g. 1.5 seconds. If no pulse is detected within any 1.5-second time interval, the threshold next below the current threshold will instead be used for pulse detection.

As a yet further alternative method, the first detection threshold to be applied when starting the detection method may be neither the highest threshold nor the lowest threshold, e.g. $AT_2$ or $AT_3$ as in the example shown in Fig. 2. In such a case, the extent of variation (e.g. as determined by the standard deviation) of the pulse intervals is constantly determined and compared with a pre-set value, and it will also be checked as to whether any pulse (i.e. QRS complex) is detected within a continuous pre-determined time interval, e.g. 1.5 seconds. If the extent of variation is larger than the pre-set value, the threshold next above the current threshold will instead be used for pulse detection, whereas if no pulse is detected within any 1.5-second time interval, the threshold next below the current threshold will instead be used for pulse detection.

It should be understood that the above only illustrates examples whereby the present invention may be carried out, and that various modifications and/or alterations may be made thereto without departing from the spirit of the invention. For example, the extent of deviation of the pulse intervals may be measured by some other means, e.g. the variance, which is the square of the standard deviation of the pulse intervals. The number of detection thresholds may, of course, also vary.

It should also be understood that certain features of the invention, which are, for clarity, described in the context of separate embodiments, may be provided in combination in a single embodiment. Conversely, various features of the invention which are, for brevity, described in the context of a single embodiment, may also be provided separately or in any appropriate sub-combinations.

**Claims**

1.    A method of measuring heart rate of an individual, including the steps of:

> (a) pre-setting a plurality of discrete detection thresholds including at least a highest threshold and a lowest threshold;
> (b) pre-setting at least a first condition for change of detection threshold;
> (c) detecting a stream of signals in an ECG waveform;
> (d) detecting QRS complexes in said ECG waveform by applying a first one of said pre-set detection thresholds; and
> (e) detecting QRS complexes in said ECG waveform by applying instead a second one of said pre-set detection thresholds which is different from said first detection threshold if said at least first condition is met in said step (d).

2. A method according to Claim 1 further **characterized in that** said first one of said pre-set detection thresholds is the highest threshold.

3. A method according to Claim 1 further **characterized in that** said first one of said pre-set detection thresholds is the lowest threshold.

4. A method according to Claim 1 further **characterized in that** said second one of said pre-set detection thresholds is adjacent to said first one of said pre-set detection thresholds.

5. A method according to Claim 4 further **characterized in that** said second one of said pre-set detection thresholds is next above said first one of said pre-set detection thresholds.

6. A method according to Claim 4 further **characterized in that** said second one of said pre-set detection thresholds is next below said first one of said pre-set detection thresholds.

7. A method according to Claim 1 further **characterized in that** said first condition is that no QRS complex is detected within a continuous pre-determined time interval.

8. A method according to Claim 7 wherein said pre-determined time interval is substantially 1.5 seconds.

9. A method according to Claim 1 further **characterized in that** said first condition is that the extent of variation of time intervals of successive detected QRS complexes is larger than a pre-set value.

10. A method according to Claim 9 further **characterized in** including a step (f) of determining the extent of variation of time intervals between successively detected QRS complexes.

11. A method according to Claim 10 further **characterized in that** said step (f) is carried out by determining the standard deviation of said time intervals.

12. A method according to Claim 1 further **characterized in that**, in said step (b), at least a second condition for change of detection threshold is also pre-set.

13. A method according to Claim 12 further **characterized in that** said second one of said pre-set detection thresholds is next below said first one of said pre-set detection thresholds if said first condition is met, and said second one of said pre-set detection thresholds is next above said first one of said pre-set detection thresholds if said second condition is met.

14. A method according to Claim 7 further **characterized in** including a step (g) of detecting QRS complexes in said ECG waveform by applying instead the highest threshold if no QRS complex is detected within said pre-determined period of time by applying the lowest threshold.

15. A method according to Claim 1 further **characterized in that** said thresholds are amplitude thresholds.

16. A device for measuring heart rate of an individual, including:

   means for pre-setting a plurality of discrete detection thresholds including at least a highest threshold and a lowest threshold; and
   means for detecting a stream of signals in an ECG waveform;
   wherein said device is adapted to detect QRS complexes in said ECG waveform by applying a first one of said pre-set detection thresholds;
   **characterized in that** said device is adapted to detect QRS complexes in said ECG waveform by applying instead a second one of said pre-set detection thresholds which is different from said first detection threshold if at least a first pre-set condition is met.

17. A device according to Claim 16 further **characterized in that** said first one of said pre-set detection thresholds is the highest threshold.

**18.** A device according to Claim 16 further **characterized in that** said first one of said pre-set detection thresholds is the lowest threshold.

**19.** A device according to Claim 16 further **characterized in that** said second one of said pre-set detection thresholds is adjacent to said first one of said pre-set detection thresholds

**20.** A device according to Claim 19 further **characterized in that** said second one of said pre-set detection thresholds is next above said first one of said pre-set detection thresholds.

**21.** A device according to Claim 19 further **characterized in that** said second one of said pre-set detection thresholds is next below said first one of said pre-set detection thresholds.

**22.** A device according to Claim 16 further **characterized in that** said first condition is that no QRS complex is detected within a continuous pre-determined time interval.

**23.** A device according to Claim 22 wherein said pre-determined time interval is substantially 1.5 seconds.

**24.** A device according to Claim 16 further **characterized in that** said first condition is that the extent of variation of time intervals of successive detected QRS complexes is larger than a pre-set value.

**25.** A device according to Claim 24 further including means for determining the extent of variation of time intervals between successively detected QRS complexes.

**26.** A device according to Claim 25 further **characterized in that** said determining means is adapted to determine the standard deviation of said time intervals.

**27.** A device according to Claim 16 further **characterized in that** said second one of said pre-set detection thresholds is next below said first one of said pre-set detection thresholds if said first condition is met, and said second one of said pre-set detection thresholds is next above said first one of said pre-set detection thresholds if a second condition is met.

**28.** A device according to Claim 22 further **characterized in that** said device is adapted to detect QRS complexes in said ECG waveform by applying instead the highest threshold if no QRS complex is detected within said pre-determined period of time by applying the lowest threshold.

**29.** A device according to Claim 16 further **characterized in that** said thresholds are amplitude thresholds.

**30.** A device according to Claim 16 further **characterized in that** said detecting means includes a level comparator.

**31.** A device according to Claim 16 further **characterized in that** said detecting means includes a QRS detection circuit.

Fig. 1

$C_1$    $C_2$

$AT_1$

$AT_2$

$AT_3$

$AT_4$

P-Wave

QRS
Complex

T-Wave

Pulses
Detected

T

<u>Fig. 2</u>

ECG → INA (200) → Filter/Autocorrelation (202) → QRS Detection (204) —R-R Intervals→ Heart Rate Calculation (206) → HR

Fig. 3

ECG → INA (220) → Filter/Autocorrelation (222) → Level comparator (224) —Pulses→ Heart Rate Calculation (226) → HR

Fig. 4

246    250                                              252

MCU

ECG → INA → Filter → ADC → Level comparator → Pulses → Heart Rate Calculation → HR

240    242                                    248

Threshold adjustment

## Fig. 5

268    266

MCU

ECG → INA → Filter → Level comparator → Pulses → Heart Rate Calculation → HR

260    262    264

Threshold

Threshold adjustment

Electronic Switching Network

270

## Fig. 6

300

302

Start

Set the threshold
to the highest level

Reset Buffer in the
HR calculation — 304

314 — Set threshold to
next higher level

306

Pulse detected
within 1.5
seconds?

Yes

No

Variation of
pulse interval
too large?

Yes

312

No

308

Threshold
currently at the
lowest level?

Yes

No

310

Set threshold to
next lower level

Fig. 7

**European Patent Office**

**EUROPEAN SEARCH REPORT**

Application Number

EP 05 25 6339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 891 048 A (NIGAM ET AL) 6 April 1999 (1999-04-06)<br><br>* figure 1 *<br>* figures 3a-3d *<br>* column 6, lines 4-40 *<br>* column 7, lines 9-23 *<br>* column 9, lines 4-62 *<br>* column 12, lines 26-33 * | 1-8, 15-23, 29-31 | A61B5/0456 G06F17/00 |
| Y | | 9-11,14, 24-26,28 | |
| X | US 5 103 819 A (BAKER ET AL) 14 April 1992 (1992-04-14)<br><br>* figure 2 *<br>* figure 3 *<br>* figure 5 *<br>* figure 9 *<br>* column 3, lines 29-36 *<br>* column 4, lines 20-24 *<br>* column 5, lines 6-53 *<br>* column 6, lines 28-68 *<br>* column 7, lines 1-5 *<br>* column 8, lines 25-27 *<br>* column 10, lines 1-12 * | 1-6,12, 13, 16-21, 27,30,31 | |
| Y | | 9-11,14, 24-26,28 | TECHNICAL FIELDS SEARCHED (IPC)<br><br>A61B G06F A61N |
| Y | WO 2004/093974 A (MEDTRONIC, INC; GUNDERSON, BRUCE, D) 4 November 2004 (2004-11-04)<br>* figure 9 *<br>* page 9, lines 20-27 *<br>* page 23, lines 16-25 *<br>* page 25, lines 3-5 *<br>-----<br>-/-- | 9-11, 24-26 | |

The present search report has been drawn up for all claims

| Place of search | Date of completion of the search | Examiner |
|---|---|---|
| Munich | 30 March 2006 | Gentil, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent**
**Office**

**EUROPEAN SEARCH REPORT**

Application Number

EP 05 25 6339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 4 938 228 A (RIGHTER ET AL) 3 July 1990 (1990-07-03) * figure 8 * * column 10, lines 8-11 * ----- | 14,28 | |
| | | | TECHNICAL FIELDS SEARCHED (IPC) |

The present search report has been drawn up for all claims

| Place of search | Date of completion of the search | Examiner |
|---|---|---|
| Munich | 30 March 2006 | Gentil, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................
& : member of the same patent family, corresponding document

EPO FORM 1503 03.82 (P04C01)

**EP 1 774 907 A1**

## ANNEX TO THE EUROPEAN SEARCH REPORT
## ON EUROPEAN PATENT APPLICATION NO.

EP 05 25 6339

30-03-2006

| Patent document cited in search report | | Publication date | Patent family member(s) | | Publication date |
|---|---|---|---|---|---|
| US 5891048 | A | 06-04-1999 | DE 19626353 A1 | | 02-01-1998 |
| | | | EP 0813891 A2 | | 29-12-1997 |
| US 5103819 | A | 14-04-1992 | NONE | | |
| WO 2004093974 | A | 04-11-2004 | CA 2522672 A1 | | 04-11-2004 |
| | | | EP 1615693 A2 | | 18-01-2006 |
| US 4938228 | A | 03-07-1990 | AU 638648 B2 | | 01-07-1993 |
| | | | AU 5197990 A | | 05-09-1990 |
| | | | CA 2046870 A1 | | 16-08-1990 |
| | | | EP 0458896 A1 | | 04-12-1991 |
| | | | GB 2228331 A | | 22-08-1990 |
| | | | WO 9009144 A1 | | 23-08-1990 |

EPO FORM P0459